# EUROPÄISCHE PATENTANMELDUNG

(11) **EP 0 856 279 A2**
(43) Veröffentlichungstag der Anmeldung: **05.08.1998**
(21) Anmeldenummer: 98250043.1
(22) Anmeldetag: 04.02.1998
(51) Int. Cl.: A61B 5/00, A63B 21/00

(54) **Vorrichtung zum Biofeedback-Training**

(30) Priorität: 04.02.1997 DE 19706042
(71) Anmelder: Steindorf, Marianne, 27777 Ganderkesee (DE)
(72) Erfinder: Steindorf, Jürgen W., 27777 Ganderkesee (DE)
(74) Vertreter: Müller, Wolfram Hubertus, Dipl.-Phys.

(57) **Zusammenfassung**

Vorrichtung zum Biofeedback-Training der Muskulatur mit einer optischen und/oder akustischen Anzeigeeinrichtung, einer Einrichtung zur Eingabe von Sollwerten für Trainingsparameter, einem Anschluß für einen Istwert- oder Meßwertaufnehmer zur Erfassung des elektromyographischen Muskelpotentials oder des Muskel-Kontraktionsdruckes und einem Datenspeicher zur Speicherung der Ist-, Soll- und Meßwerte der Trainingsparameter, von Zeit- und Zählparametern und eines Auswertungsprogramms. In einer Istwertmessung wird ein Ruhetonus bei entspannter Muskulatur, eine im wesentlichen maximale Kontraktion und eine Ist-Haltezeit, über die die maximale Kontraktion gehalten wird, erfaßt und ein Sollwert für die Trainingskontraktion als prozentualer Wert der um den Ruhetonus reduzierten Maximalkontraktion und ein Sollwert für die Trainingshaltezeit als prozentualer Wert der Ist-Haltezeit eingegeben. Vor jeder Trainingseinheit wird der Ruhetonus erfaßt und bei jeder Trainingseinheit (Sitzung) werden die Istwerte der Trainingsparameter erfaßt, mit den Sollwerten verglichen, ausgewertet und optisch und/oder akustisch ausgegeben.

## Beschreibung

Die Erfindung betrifft eine Vorrichtung zum Biofeedback-Training nach dem Oberbegriff des Anspruchs 1.

Eine derartige Vorrichtung ist aus der DE 195 08 735 A1 bekannt. Sie wendet das Biofeedback als Methode an, physiologische Abläufe, die im wesentlichen im vegetativen Nervensystem ablaufen und daher nicht oder nur sehr ungenau durch die Sinnesorgane erfaßt werden, der bewußten Wahrnehmung zugänglich zu machen und gezielt zu beeinflussen, insbesondere für therapeutisch gesetzte Ziele. Dabei werden die betreffenden Körperfunktionen registriert und dem Patienten optisch und/oder akustisch angezeigt, so daß der Patient lernt, sein Verhalten während der Therapie gezielt zu verändern.

Das Biofeedbacktraining wird insbesondere auch zur Behandlung der Anal- und Urinalinkontinenz angewandt. Dabei üben die Patienten mehrfach die Kontraktion der Muskelgruppen (M. levator ani, M. puborectalis, M. sphinkter ani ext.). Deren Aktivitäten werden als elektrisches Summenpotential oder als Druckprofil registriert und dem Patienten optisch und/oder akustisch zurückvermittelt. Ziel der Therapie ist eine Kräftigung der Muskulatur, insbesondere der aeroben und anaeroben Kraftausdauer, sowie die Steigerung der eigenen Wahrnehmungsfähigkeit.

Bei der aus der DE 195 08 735 A1 bekannten Vorrichtung zum Biofeedback-Training werden muskelphysiologische Erkenntnisse angewandt, die einem kontrollierten, isometrischen Krafttraining der betreffenden Muskulatur, insbesondere der Beckenboden- und Sphinctermuskulatur zur Inkontinenz-Therapie, sowie der Verbesserung der Sensorik dienen. Dabei wird das Trainingsprogramm nicht nur unter Berücksichtigung der Kontraktionskraft durchgeführt, sondern auch unter Berücksichtigung der Kontraktionsdauer und des Ruhetonus, d.h. der im entspannten Zustand des Patienten latent vorhandenen Muskelkontraktion. Um den Trainingseffekt unter Berücksichtigung einer morphologischen und funktionellen Anpassung zur Leistungssteigerung so optimal wie möglich zu gestalten, werden die Trainingsparameter für jeden Patienten individuell ermittelt und für das Training programmiert.

Zu diesem Zweck wird vor dem Training eine Messung
a) des Ruhetonus (Ruhewert, Ruhekontraktion) des Muskels bei entspannter Muskulatur;
b) der maximalen Kontraktionskraft und
c) der Ist-Haltezeit, in der die maximale Kontraktion gehalten wird
durchgeführt. Diese Parameter können annähernd automatisch ermittelt werden.

Aus der Istwertmessung, und aus den Parametern a) bis c), wird die Sollwertvorgabe für die aufzubringende Kraft "Trainingskontraktion", und die Sollwertvorgabe "Trainingshaltezeit" gebildet. Die "Trainingskontraktion" ist die Differenz aus der Maximalkontraktion und dem Ruhetonus, gewichtet mit einem Faktor, der gemäß muskelphysiologischer Trainingsgrundlagen eine optimale Trainingsbelastung ergibt. Die Trainingshaltezeit ergibt sich aus der Gewichtung der Isthaltezeit mit einem Faktor, der gemäß muskelphysiologischer Trainingsgrundlagen eine optimale Trainingsbelastung bewirkt. Die Trainingsparameter können graphisch und/oder numerisch in einer Anzeigeeinrichtung dargestellt und bearbeitet werden

Zusätzlich zu den Traingingsparametern "Trainingskontraktion" und "Trainingshaltezeit" wird die Anzahl der aus einer Kontraktions- und einer Entspannungsphase bestehenden Zyklen, d.h. die Anzahl der Wiederholungen in einer Trainingssitzung, das Pausenzeitverhältnis, d.h. das Verhältnis der Zeit zwischen Kontraktions- und Entspannungsphase, sowie die Anzahl der Sitzungstage (Sequenz länge) bis zum Aktivieren der nächsten Sequenz vorgegeben. Zur Bewertung des Trainings wird die in einer Sitzung erreichte Trainingshaltezeit und der erreichte Trainingskontraktionswert aus den Mittelwerten (Kraft und Zeit) der Trainingszyklen errechnet und der erreichte prozentuale Anteil bezogen auf die Trainingssollwerte gespeichert. Das gespeicherte Trainingsergebnis wird bei der automatischen Anpassung der Trainingsparameter berücksichtigt und nach Art einer Siegerehrung optisch dargestellt bzw. durch eine akustische Meldung angegeben.

Bei der praktischen Erprobung der bekannten Vorrichtung wurde festgestellt, daß der Ruhetonus sehr stark vom Erregungszustand des Patienten bzw. Trainierenden abhängt und demzufolge von Sitzung zu Sitzung stark schwanken und dabei wesentlich von dem bei der Istwert-Messung vor Aufnahme des Trainingsprogramms abweichen kann. Dies führt dazu, daß der Patient während einer Sitzung oder bei mehreren Sitzungen einer Trainingssequenz ständig unterfordert oder ständig überfordert ist, so daß das mit der Sollwertvorgabe der Trainingsparameter gesteckte Ziel einer optimalen Leistungssteigerung nicht erreicht wird. Dies führt zum Teil zu erheblichen Verzögerungen beim Muskelaufbau und damit zu einer Minderung der Effizienz der bekannten Vorrichtung.

Aufgabe der vorliegenden Erfindung ist es, die Trainingsparameter während einer Sitzung an den aktuellen Trainingszustand eines Patienten anzupassen.

Diese Aufgabe wird erfindungsgemäß durch die Merkmale des Anspruchs 1 gelöst.

Mit der erfindungsgemäßen Lösung wird der dem jeweiligen Entspannungszustand bei Antritt einer Sitzung entsprechende aktuelle Ruhetonuswert erfaßt und eine Anpassung an den jeweiligen Entspannungszustand des Trainierenden durch eine Nachführung des Ausgangspunktes des Trainingsparameters "Trainingskontraktion" vorgenommen.

Vorzugsweise wird vor jeder Trainingssequenz eine Entspannungsphase durchgeführt, in der der Ruhetonus erfaßt und optisch und/oder akustisch ausgegeben wird.

Eine dem eigentlichen Biofeedback-Training vorangehende Entspannungsphase dient dazu, den Patienten vor jeder Sitzung zu beruhigen, um möglichst immer den gleichen Ausgangsstatus bezüglich des Ruhepotentials (-tonus) für das aufzubringende Trainingspotential zu erhalten, da nicht davon ausgegangen werden kann, daß ein Patient in jeder Sitzung das gleiche Ruhepotential besitzt. Äußere Einflüsse können das Ruhepotential bzw. den Ruhetonus von Sitzung zu Sitzung erheblich differieren lassen und damit zu einer Verfälschung des Trainingsergebnisses führen, wenn beispielsweise die vorgegebene Trainingskontraktion erreicht wird, die der Differenz zwischen dem erreichten Kontraktionswert und dem Ruhetonus aber aufgrund eines gegenüber der Istwertmessung höheren Ruhetonus geringer ist als bei der dem Trainingsprogramm zugrundegelegten Istwertmessung.

Eine vorteilhafte Ausgestaltung der erfindungsgemäßen Lösung ist dadurch gekennzeichnet, daß die Entspannungsphase in einer vorgegebenen Zeitspanne durchgeführt wird.

Da das Biofeedback-Training nicht nur die willkürliche Kontraktion der zu trainierenden Muskeln auch deren zu trainierenden Muskelpartien vorsieht, sondern auch deren Entspannung, wird vorteilhafterweise ein zeitlicher Rahmen für die Entspannungsphase vorgegeben, innerhalb der der Trainierende nach Möglichkeit auf den gleichen Ruhetonus zurückkehren soll wie bei der dem Trainingsprogramm vorangegangenen Sollwertermittlung festgestellt wurde.

Durch Vorgabe eines einstellbaren Toleranzbereiches oberhalb und unterhalb des in der Istwertmessung erfaßten Ruhetonus werden geringfügige Schwankungen des Ruhepotentials in der Entspannungsphase eliminiert, weil diese geringfügigen Abweichungen nicht das Trainingsziel beeinflussen und auch auf Meßtoleranzen zurückgeführt werden können.

Durch Wiederholung der Entspannungsphase in den Fällen, wo der bei Sitzungsbeginn erfaßte Ruhetonus außerhalb des vorgegebenen Toleranzbereiches liegt, können kurzfristige Erregungszustände des Trainierenden ausgeschaltet und das Trainingsprogramm entsprechend dem vorgegebenen Programmablauf weitergeführt werden.

Liegt auch nach einer einstellbaren Anzahl von Wiederholungen der Entspannungsphase der erfaßte Ruhetonus außerhalb des vorgegebenen Toleranzbereiches, so wird in Abhängigkeit des aktuellen Ruhepotentials das Toleranzfeld, und damit der Trainingskontraktionswert, in vorgebbaren Schritten angehoben und gesenkt. Würde das Training im Anschluß an die Entspannungsphase ohne Anpassung der eben genannten Werte durchgeführt werden, wäre der Trainierende entweder mühelos in der Lage die gewünschte Kontraktionskraft zu übertreffen, oder er würde das durch den Trainingsparameter "Trainingskontraktion" vorgegebene Ziel nicht erreichen.

Nach einer weiteren Ausgestaltung der erfindungsgemäßen Lösung kann die Entspannungsphase in einer Sitzung mehrfach wiederholt werden. Wird auch bei den Wiederholungen das Toleranzfeld einer Anpassung über- oder unterschritten, so wird eine Warnmeldung ausgegeben und der Fehlversuch protokolliert.

Vorzugsweise wird die Entspannungszeit durch die optische Darstellung einer ablaufenden Uhr wiedergegeben und der Ruhetonus nach Art einer Füllstandsanzeige angegeben, so daß der Trainierende anhand des Füllstandes einer Säule seine Anspannung erkennen kann. Gleichzeitig läuft die dargestellte Uhr, die sinnbildlich die Entspannungszeit repräsentiert. Liegt der Mittelwert des in der Entspannungsphase erfaßten Ist-Ruhepotentials außerhalb des Toleranzbereiches des Soll-Ruhetonus, wird nach den vorangegangenen Merkmalen die Entspannungsphase wiederholt.

Um dem Trainierenden etwas Zeit zur Entspannung zu geben und um den Mittelwert des erfaßten Ist-Ruhepotentials nicht zu sehr zu verwischen, werden nur die Potentialwerte im letzten Viertel der Vorlaufzeit gesammelt und weiterverarbeitet.

Durch eine automatische Parameteranpassung werden Leistungssteigerungen des Trainierenden berücksichtigt und optimal zum Erreichen des Trainingsziels angewandt. Dazu werden die Trainingsergebnisse der einzelnen Sitzungen mit ihren mehreren Zyklen erfaßt und die an mehreren Tagen durchgeführten Sitzungen entsprechend einer vorgegebenen Sequenzlänge ausgewertet. Wurden die Trainingsparameter an allen Trainingstagen einer Sequenz länge vollständig erfüllt, wird automatisch die nächste Trainingssequenz mit der einstellbaren Anzahl von Trainingstagen aktiviert und die entsprechenden Trainingsparameter der betreffenden Trainingssequenz eingesetzt. Dabei werden nur die Sitzungen ausgewertet, die an verschiedenen Tagen durchgeführt wurden, um zu verhindern, daß mehrere an einem Tag durchgeführte Sitzungen zum Aktivieren der nächstfolgenden Trainingssequenz führen. Vorrichtung nach mindestens einem der vorhergehenden Ansprüche, dadurch gekennzeichnet, daß eine annähernd automatische Bestimmung der Parameter Maximalkraft, Ruhetonus, Isthaltezeit und Trainingsparameter "Trainingskraft" und "Trainingshaltezeit" aus der Messung für die Parameterfestlegung vorgenommen werden kann.

Um eine Unter- oder Überforderung im Training zu vermeiden, wird nach einem weiteren Merkmal der Erfindung bei Über- oder Unterschreiten eines Toleranzfeldes das Training automatisch abgebrochen.

Nach einem weiteren Erfindungsmerkmal wird eine grobe Fehlbedienung dadurch vermieden, daß durch eine logische, konsequente, optische und akustische Bedienerführung Fehler seitens des Bedieners ausgeschlossen werden.

Anhand eines in der Zeichnung dargestellten Ausführungsbeispieles soll der Erfindung zugrunde liegende Gedanke näher erläutert werden. Es zeigen:
- Fig. 1: eine Hüllkurvendarstellung einer Istwertmessung der Kontraktionskraft und Haltezeit, und die Markierung der Trainingsparameter;
- Fig. 2: die zur Hüllkurvendarstellung gemäß Figur 1 gehörende Istwertmessung;
- Fig. 3: eine schematische Darstellung eines Parametermenüs mit automatischer Parameteranpassung;
- Fig. 4: ein Flußdiagramm eines Trainingsablaufs;
- Fig. 5: eine bildliche Darstellung der Meßwerte in einer Entspannungsphase;
- Fig. 6: ein Flußdiagramm der Vorlaufphase;
- Fig. 7: eine schematische Darstellung des zeitlichen Ablaufs eines Trainingszyklus zur Erläuterung der verschiedenen Bereiche;
- Fig. 8: eine zeitliche Darstellung der Bewertung Ablaufs eines Trainingszyklus.

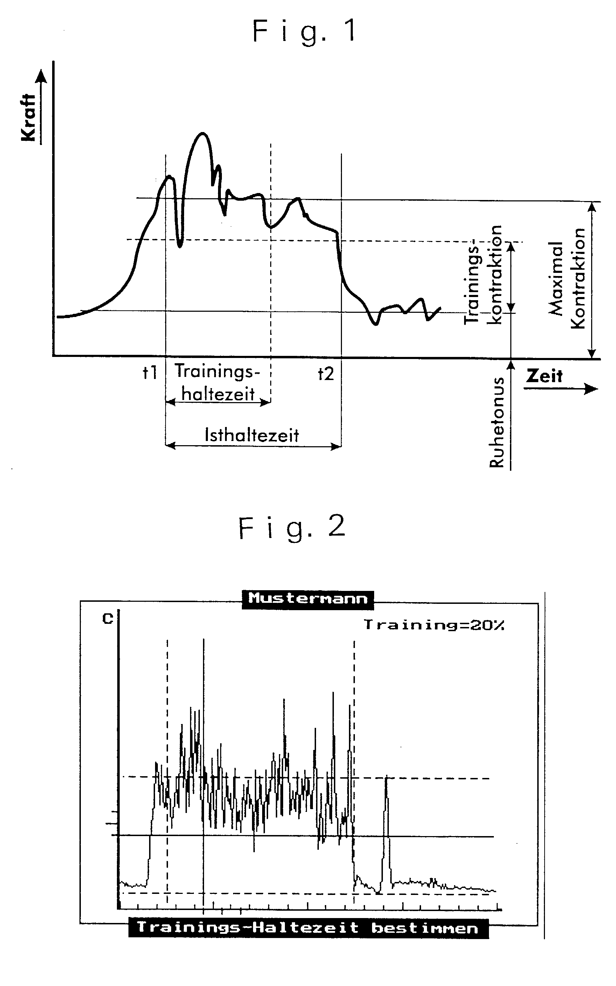

Die Figuren 1 und 2 zeigen ein Beispiel einer Istwertmessung und Hüllkurvendarstellung der Kontraktionskraft über der Zeit. Der Meßvorgang wird nach Anlegen bzw. Einführen der Meßsonden ausgelöst und der Patient bzw. Trainierende dazu angehalten, die betreffenden Muskeln bzw. die Muskelpartie so kräftig wie möglich zu betätigen. Mit steigender Muskelkontraktion wird an der Meßsonde entweder ein höheres elektromyographisches Potential oder ein erhöhter Druck je nach Art der Meßsonde bewirkt.

Gleichzeitig wird der Trainierende dazu angehalten, die ihm mögliche maximale Kontraktionskraft so lange wie möglich zu halten.

Das Ergebnis eines derartigen Meßvorganges ist in den Figuren 1 und 2 beispielhaft dargestellt und zeigt, daß die Kontraktionskraft ausgehend von einem Ruhetonus ansteigt und schließlich nach Erreichen eines Höchstwertes wieder auf den Ruhetonus zurückfällt.

Mit Hilfe eines Auswertungsprogramms wird der Ruhetonus, die Maximalkontraktion, und die Ist-Haltezeit festgelegt. Diese Parameter können vom Programm annähernd automatisch ermittelt werden, und sind vom Bediener nachträglich veränderbar.

Im Ruhetonus sind die Muskeln bzw. die Muskelpartie zwar entspannt, verhalten sich in diesem Zustand aber elektrisch nicht neutral. Da verschiedene Muskelzellen gegeneinander arbeiten, ist stets ein Ruhepotential vorhanden, das von der Tageszeit, psychischen Verfassung, Menstruationszyklen und unmittelbar vorhergehende Bewegungsaktivitäten abhängt. Unter Ruhetonus versteht man daher das Potential des betreffenden Muskels im Entspannungszustand.

Zur manuellen Bestimmung des Ruhepotentials bzw. Ruhetonus erscheint am unteren Rand der aufgenommenen Meßkurve eine parallel zur Zeitachse verlaufende Cursormarkierung, die auf die Höhe des Ruhepotentials gebracht wird. Nachfolgend erscheint am oberen Rand eine weitere Cursormarkierung, mit der die Maximalkontraktion festgelegt wird. Auf der Zeitachse wird über zwei bewegliche, parallel zur Kraftachse verlaufende, Cursobegrenzungen die Haltezeit manuell eingestellt.

Als Isthaltezeit wir die Zeitspanne genommen, in der die Maximalkontraktion gedanklich auf einer Plateauhöhe gehalten wird. Nach der zeitlichen Darstellung der Kontraktionskraft gemäß Fig. 1 verläuft die Haltezeit vom Zeitpunkt t1 bis zum Zeitpunkt t2.

Ferner ist die Ermittlung der Maximalkontraktion sowie der Haltezeit automatisch basierend auf der aufgenommen Meßkurve rechnergestützt implementiert. Diese berechneten Werte können vom Bediener manuell nachgearbeitet werden.

Anhand dieser Istwerte werden als Trainingsparameter die Trainingskontraktion und die Trainingshaltezeit bestimmt. Dies erfolgt entweder automatisch durch eine rechnergestützte Vorgabe oder halbautomatisch durch Markierung eines Verstellbereiches, innerhalb dessen die Trainingskontraktion und die Trainingshaltezeit als Bruchteil der Maximalkontraktion und der Isthaltezeit als Sollwert für das Trainingsprogramm manuell vorgegeben oder verändert werden können.

Die Trainingskontraktion kann automatisch, halbautomatisch oder manuell dadurch bestimmt werden, daß mit einem parallel zur Zeitachse verlaufenden Cursor eine Markierung gesetzt wird, die beispielsweise zwischen 50% und 70% der Differenz aus maximaler Kontraktion und Ruhetonus liegen kann. Entsprechende %-Marken befinden sich auf der Kraftachse.

Anschließend wird die Trainingshaltezeit bestimmt, die beispielsweise zwischen 20% und 40% der Isthaltezeit liegt. Auch dieser Trainingsparameter kann automatisch oder halbautomatisch durch entsprechende Cursormarkierungen auf der Zeitachse festgelegt werden, so daß eine durchgezogene Cursormakierung parallel zur Kraftachse in diesem Bereich bewegt werden kann.

Nach der Auswertung der Messung werden die Trainingsparameter zusammen mit der Meßwertkurve in einen Datensatz zusammen mit personenbezogenen Daten des Trainierenden bzw. Patienten abgelegt. Die Messung kann daher jederzeit nachträglich bearbeitet werden.

Da sich eine Sitzung aus mehreren Trainingszyklen mit jeweils einer Kontraktions- und einer Entspannungsphase zusammensetzt und mehrere Sitzungen erforderlich sind, um die Trainingsparameter der Leistungsfähigkeit des Trainierenden anzupassen, sind als weitere Trainingsparameter die Zyklenzahl, d.h. die Anzahl der Wiederholungen eines Trainingsschemas in einer Sitzung, das Pausenzeitverhältnis, d.h. das Verhältnis zwischen Kontraktions- und Entspannungsphase sowie die Sequenz länge (Anzahl der Sitzungstage) festzulegen, die die Anzahl der Sitzungen bzw. Trainingstage einer Sequenz bis zur Aktivierung der nächsten Sequenz kennzeichnet.

Diese Trainingsparameter können für eine nahezu beliebige Anzahl Sequenzen vorprogrammiert werden, so daß bei Erfüllung der vorgegebenen Anzahl der Sitzungen und bei einem vollständigen Erfüllen der Trainingsparameter bzw. Vorgaben an allen Trainingstagen die jeweils nächste Sequenz aktiviert wird. Sitzungen bzw. Trainingstage, an denen die Vorgaben nicht vollständig errreicht wurden, werden nicht gezählt, so daß sich der Zeitpunkt des zur Aktivierung der nächstfolgenden Sequenz entsprechend verschiebt.

Für ein optimal leistungssteigerndes Programm werden nicht die Kalendertage, sondern die tatsächlich durchgeführten Trainingstage erfaßt und bei Durchführung von mehreren Sitzungen an einem Tag diese nur zu einem Trainingstag gezählt.

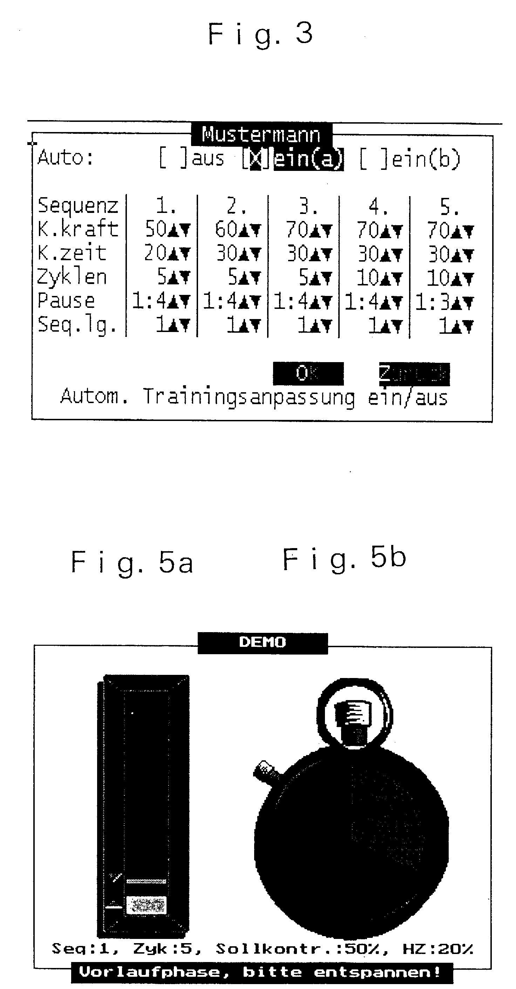

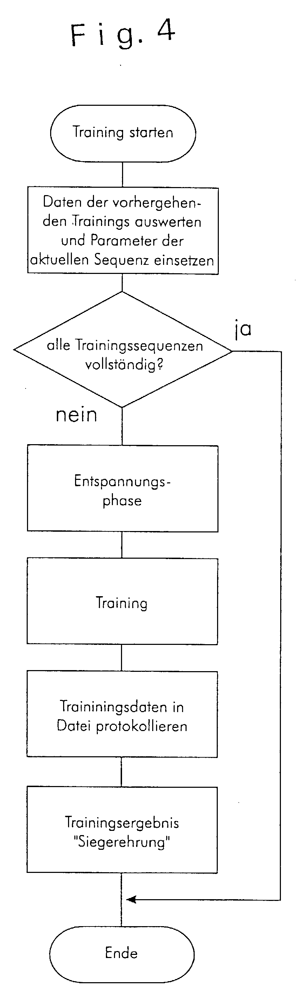

Fig. 3 zeigt ein schematisches Parametermenü, mit dessen Hilfe die verschiedenen Trainingsparameter für in diesem Beispiel 5 Sequenzen eingestellt werden können. Die schwarzen Dreiecke bezeichnen die Verstellung der Werte zu höheren bzw. niedrigeren Werten.

Bei der Durchführung des Trainings innerhalb einer Sitzung wird zwischen einer Entspannungsphase und der Kontraktionsphase unterschieden. Nach dem Start des Trainings werden entsprechend dem Flußdiagramm gemäß Fig. 4 zunächst aus der Trainingsdatei des Trainierenden alle vorhergehenden Sitzungen einer oder mehrerer Trainingssequenzen ausgewertet.

Wurden alle Trainingstage einer Sequenz länge vollständig erfüllt, wird die nächste Trainingssequenz aktiviert und deren Trainingsparameter werden entsprechend der automatischen Anpassung eingesetzt.

Wurden alle Trainingstage der letzten Trainingssequenz erfolgreich durchgeführt, dann wird das Trainingsprogramm mit einer optischen/akustischen Meldung beendet. Ohne Veränderung der Trainingsparameter durch den Arzt oder Therapeuten kann das Trainingsprogramm für diesen Trainierenden bzw. Patienten dann nicht erneut gestartet werden.

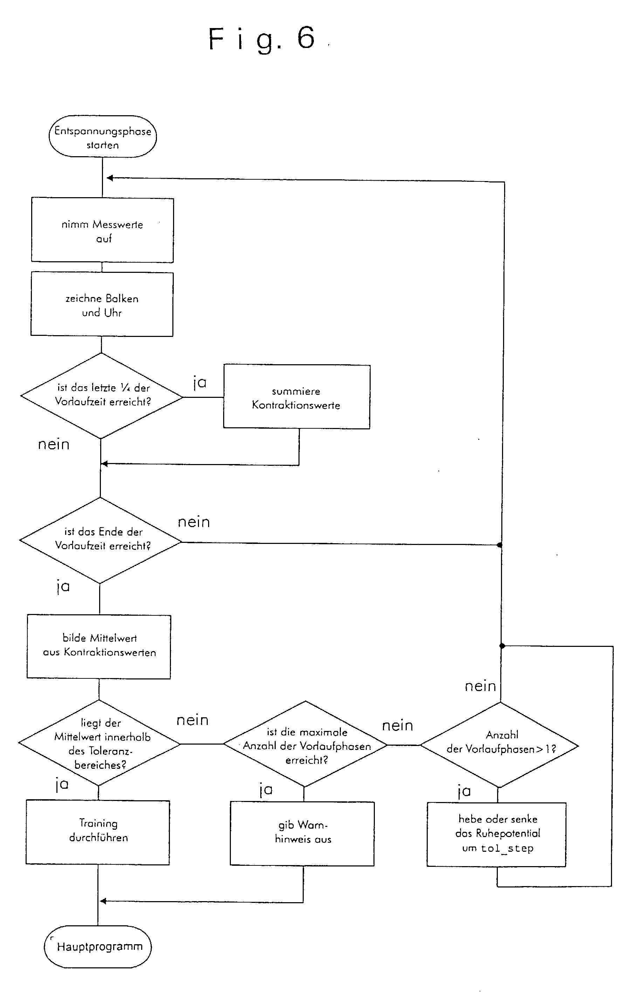

Ist die Anzahl der Sequenzen noch nicht vollständig, folgt eine Vorlaufphase, die den Trainierenden bzw. Patienten zu jeder Sitzung beruhigen soll, um möglichst immer den gleichen Ausgangsstatus zu erhalten. Wurde beispielsweise die Sollwertermittlung an einem Tag durchgeführt, an dem der Trainierende konditionell gut war, so orientieren sich alle Trainingsparameter entsprechend der vorstehenden Darstellung an dieser Sollwertermittlung. Nimmt der Trainierende dann an einem Folgetag das Trainingsprogramm auf, dann erreicht er unter Umständen diese Trainingsvorgaben entsprechend den ausgewählten Trainingsparametern nicht. Zu diesem Zweck wird vor dem eigentlichen Training eine Entspannungsphase durchgeführt, in der eine Anpassung der Toleranzfeldlage und damit der Kontraktionssollwertvorgabe vorgenommen wird, was nachfolgend anhand der Fig. 6 erläutert werden soll.

Nach der Vorlaufphase folgt das eigentliche Training, in dem mehrere Trainingszyklen durchgeführt werden. Die Trainingsergebnisse werden gesammelt und aus den Mittelwerten der während einer Sitzung erreichten Haltezeiten und Kontraktionspotentiale das Trainingsergebnis gespeichert. Nach Auswertung und Bewertung des Trainingsergebnisses wird anhand eines Bewertungsschemas eine Einstufung vorgenommen und über eine "Siegerehrung" angezeigt, die dem Trainierenden eine Selbsteinschätzung ermöglichen soll.

In der dem Training vorangehenden Entspannungsphase soll der Trainierende bzw. Patient ruhiggestellt werden, da nicht davon ausgegangen werden kann, daß ein Patient in jeder Sitzung bzw. Sitzung das gleiche Ruhepotential besitzt. Äußere Einflüsse können das Ruhepotential von Sitzung zu Sitzung erheblich schwanken lassen.

Fig. 5 zeigt eine grafische Darstellung, mit deren Hilfe die Entspannungsphase festgelegt und das Ruhepotential bzw. der Ruhetonus bestimmt wird.

Fig. 5a zeigt die in der Entspannungsphase erfaßte Kontraktionskraft, die nach Art einer Füllstandssäule aufgebaut ist. Anhand des Füllstandes der Säule kann der Patient seine Anspannung erkennen. Gleichzeitig läuft eine in Fig. 5b dargestellte Uhr, die sinnbildlich die Entspannungszeit andeutet. Da das Biofeedback-Training nicht nur die willkürliche Kontraktion des zu trainierenden Muskels vorsieht, sondern auch dessen Entspannung, wird ein zeitlicher Rahmen von beispielsweise 60 Sekunden für die Entspannungsphase vorgegeben, der aber mehrfach überschritten werden kann.

Die Füllstandsanzeige zeigt den zuvor in einer Meßwertbearbeitung festgelegten Ruhetonus sowie nach oben und unten abgegrenzt einen Toleranzbereich. Überschreitet oder unterschreitet der Füllstand der Säule den Toleranzbereich, dann wird dies durch eine vorzugsweise farbige Kennzeichnung der Säule und/oder durch eine akustische Ausgabe markiert.

Während des letzten Viertels der durch die Uhr angezeigten Entspannungsphase werden die Potentialwerte gesammelt. Liegt der Mittelwert der erfaßten Potentiale nach Ablauf der Uhr außerhalb des Toleranzbereichs, dann wird die Entspannungsphase wiederholt. Wird nach einer einstellbaren Anzahl von Durchgängen erneut der Toleranzbereich nicht eingehalten, dann wird der aktuelle Toleranzbereich und damit die Kontraktionssollwertvorgabe in vorgebbaren Schritten angehoben oder abgesenkt.

Diese Anpassung der Toleranzfeldlage an den Istzustand des Patienten vor Aufnahme des eigentlichen Trainingsprogramms ist erforderlich, um das Trainingsprogramm optimal durchführen zu können, da ohne Anpassung der Lage des Toleranzfeldes, und damit der Sollwertvorgabe der Trainingskontraktion, der Patient entweder mühelos in der Lage wäre, die vorgegebenen Parametereinstellungen der Trainingskontraktion und der Trainingshaltezeit zu übertreffen oder die vorgegebenen Trainingsparameter selbst unter größter Anstrengung nicht zu erreichen.

Während einer Sitzung kann die Entspannungsphase mehrfach wiederholt werden. Wird nach einer vorgegebbaren Anzahl Entspannungsphasen der Toleranzbereich trotz der o.g. Anpassung über- oder unterschritten, wird eine Warnmeldung ausgegeben und der Fehlversuch protokolliert.

Fig. 6 zeigt in einem Flußdiagramm den Ablauf der vorstehend beschriebenen Vorlaufphase.

Da der Trainierende oder Patient in der Regel nicht den medizinischen und technischen Hintergrund des Biofeedback-Trainings erkennt, wurden mit der erfindungsgemäßen Vorrichtung Analogien geschaffen, die die erfaßten Körperfunktionen einfach und schnell verständlich machen.

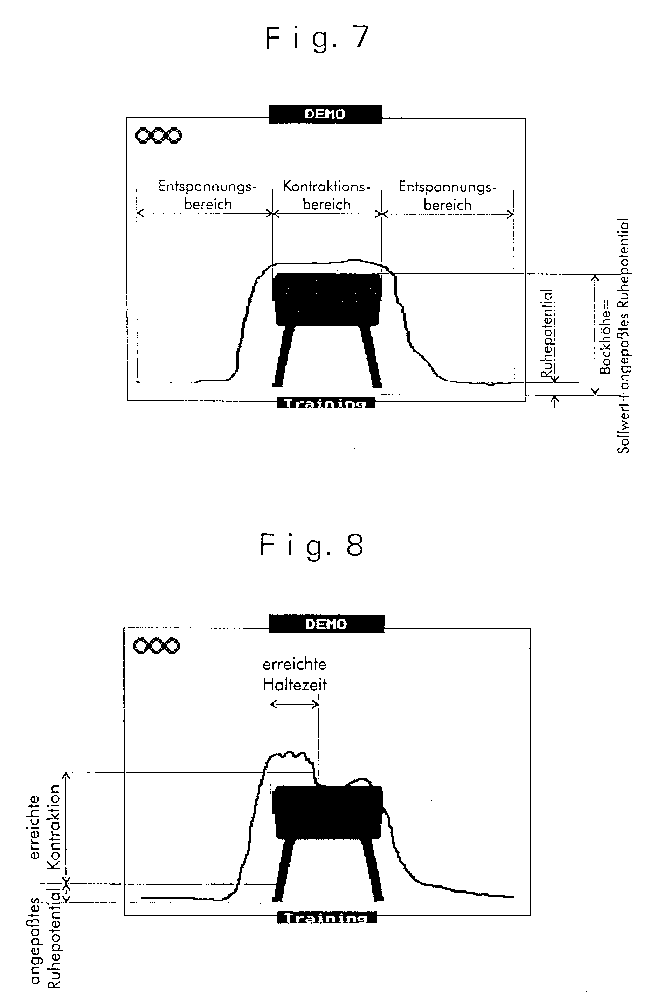

Fig 7 zeigt in einer schematischen Darstellung einen aus dem Geräteturnen bekannten Bock, dessen Höhe der in einer Traininingssitzung aufzubringenden Sollkontraktion entspricht.Von der linken Seite der bildlichen Darstellung läuft eine Linie oder ein Objekt ein, daß in der Geschwindigkeit variiert. Die Geschwindigkeit wird beispielsweise über die Trainingshaltezeit und dem Pausenzeitverhältnis bestimmt.

Da ein Trainings zyklus einer Sitzung aus einer Kontraktions- und einer Entspannungsphase besteht, wird der Trainierende in der Entspannungsphase zunächst angehalten, die Linie oder das Objekt vor dem Bock auf möglichst tiefem Niveau zu halten. In der darauffolgenden Kontraktionsphase wird der Trainierende durch Signalabgabe aufgefordert, die betreffenden Muskeln optimal zu kontrahieren, so daß das Objekt oder die Linie den Bock möglichst nicht schneidet.

Am Ende des Bockes wird ein weiteres Signal ausgegeben, woraufhin der Trainierende wieder entspannen soll. Gemäß der eingestellten Zyklenzahl wird dieser Vorgang wiederholt.

Fig. 8 zeigt die Bewertung der gemäß Fig. 7 erfaßten Kontraktionslinie, wobei zur Bewertung bestimmte Vorgaben gemacht werden. Die Trainingshaltezeit und die Trainingskontraktion werden von dem Punkt abgemessen, an dem die Kontraktionskraft den linken Bockrand und die Bockoberkante überschritten hat. Unterschreitet die Kontraktionslinie das erste Mal die obere Bockkante, dann wird die Meßwertaufnahme abgebrochen. Die Trainingshaltezeit und die Trainingskontraktion sind vollständig erfüllt, wenn die Kontraktionslinie direkt an den Rändern des Bockes verlaufen würde. Die Bockoberkante sollte vom Patienten mühelos überschritten werden können, da die Höhe maximal 70 % der aus dem Trainingsprogramm vorangegangenen Messung sein kann.

Werden während einer Sitzung die geforderten Trainingsparameter trotz eines Toleranzfeldes über- oder unterschritten, so wird das Training mit einer Warnmeldung unterbrochen, und protokolliert. Die erreichte Trainingshaltezeit und der erreichte Trainingskontraktionswert werden aus den Mittelwerten der Zyklen errechnet. Der erreichte prozentuale Anteil zur Trainingsparameter-Vorgabe wird gespeichert. Anhand einfacher Kriterien wird eine Bewertung vorgenommen, die beispielsweise nach Art einer Siegerehrung optisch/ akustisch vorgenommen werden kann.

## Patentansprüche

1. Vorrichtung zum Biofeedback-Training der Muskulatur mit einer optischen und/oder akustischen Anzeigeeinrichtung, einer Einrichtung zur Eingabe und Ausgabe von Patienten- und Diagnosedaten, einer Einrichtung zur Ermittlung und Ausgabe von Sollwerten für Trainingsparameter, einem Anschluß für einen Istwert- oder Meßwertaufnehmer zur Erfassung und Ausgabe des elektromyographischen Muskelpotentials oder des Muskel-Kontraktionsdruckprofils und einem Datenspeicher zur Speicherung der Patientendaten, der Ist-, Soll- und Meßwerte der Trainingsparameter, von Zeit- und Zählparametern und eines Auswertungsprogramms, wobei
a) in einer Istwertmessung ein Ruhetonus bei entspannter Muskulatur, eine im wesentlichen maximale Kontraktion und eine Ist-Haltezeit, über die die maximale Kontraktion gehalten wird, erfaßt, und von einem Auswertungsprogramm annähernd automatisch ermittelt werden,
b) ein Sollwert für den Trainingsparameter "Trainingskontraktion" als prozentualer Wert der um den Ruhetonus reduzierten Maximalkontraktion und ein Sollwert für den Trainingsparameter "Trainingshaltezeit" als prozentualer Wert der Ist-Haltezeit eingegeben werden,
und
c) bei jeder Sitzung die Istwerte der Trainingsparameter erfaßt, mit den Sollwerten verglichen, ausgewertet und optisch und/oder akustisch ausgegeben werden,
**dadurch gekennzeichnet**,
daß der Ruhetonus vor jeder Sitzung erfaßt und ausgewertet wird.

2. Vorrichtung nach Anspruch 1, **dadurch gekennzeichnet**, daß vor jeder Sitzung eine Entspannungsphase vorgegeben wird, in der der Ruhetonus erfaßt und optisch und/oder akustisch ausgegeben wird.

3. Vorrichtung nach Anspruch 2, **dadurch gekennzeichnet**, daß die Entspannungsphase in einer vorgegebenen Zeitspanne durchgeführt wird.

4. Vorrichtung nach mindestens einem der vorhergehenden Ansprüche, **dadurch gekennzeichnet**, daß ein einstellbarer Toleranzbereich oberhalb und unterhalb des in der Istwertmessung erfaßten Ruhetonus angezeigt wird.

5. Vorrichtung nach Anspruch 4, **dadurch gekennzeichnet**, daß die Entspannungsphase wiederholt wird, wenn der vor der Sitzung erfaßte Ruhetonus außerhalb des Toleranzbereiches liegt.

6. Vorrichtung nach Anspruch 5, **dadurch gekennzeichnet**, daß die Toleranzfeldlage und damit der Sollkontraktionswert entsprechend dem aktuellen Ruhetonuswert des Trainierenden angehoben oder abgesenkt wird, wenn nach einer vorherigen Anzahl von Wiederholungen der Entspannungsphase der erfaßte Ruhetonus außerhalb des Toleranzbereichs liegt.

7. Vorrichtung nach Anspruch 6, **dadurch gekennzeichnet**, daß die Toleranzfeldlage des Ruhetonus in vorgebbaren Schritten angehoben oder gesenkt wird.

8. Vorrichtung nach Anspruch 7, **dadurch gekennzeichnet,** daß bei einem Über- und/oder Unterschreiten der angehobenen oder abgesenkten Toleranzfeldlage des Ruhetonus in nachfolgenden Entspannungsphasen einer Sitzung eine Warnmeldung ausgegeben und ein Fehlversuch protokolliert wird.

9. Vorrichtung nach mindestens einem der vorhergehenden Ansprüche, **dadurch gekennzeichnet**, daß die Entspannungszeit innerhalb einer Entspannungsphase angezeigt wird.

10. Vorrichtung nach mindestens einem der vorhergehenden Ansprüche, **dadurch gekennzeichet**, daß der Ist-Ruhetonus in der Entspannungsphase optisch/akustisch ausgegeben wird und daß der in der Sollwertermittlung erfaßte Ruhetonus oder der in einer vorangegangenen Entspannungsphase korrigierte Soll-Ruhetonus sowie das oberhalb und unterhalb dieses Soll-Ruhetonus liegende Toleranzfeld optisch/akustisch gekennzeichnet sind.

11. Vorrichtung nach Anspruch 10, **dadurch gekennzeichnet**, daß ein Über- und Unterschreiten des Toleranzfeldes des in einer Entspannungsphase erfaßten Ist-Ruhetonus mit unterschiedlichen akustischen Meldungen oder optisch unterschiedliche Farben gekennzeichnet wird.

12. Vorrichtung nach mindestens einem der vorhergehenden Ansprüche, **dadurch gekennzeichnet**, daß eine einstellbare Zyklenanzahl für die Wiederholung eines Trainingsschemas in einer Sitzung vorgegeben wird.

13. Vorrichtung nach mindestens einem der vorhergehenden Ansprüche, **dadurch gekennzeichnet**, daß die Anzahl der Trainingstage einer oder mehrerere Sitzungen bis zur Aktivierung der nächsten Trainingssequenz vorgegeben wird.

14. Vorrichtung nach mindestens einem der vorhergehenden Ansprüche, **dadurch gekennzeichnet**, daß die eingestellten Trainingsparameter einer nachfolgenden Sequenz bei Erreichen eines vorgegebenen Trainingsziels in den vorangegangenen Sitzungen aktiviert werden.

15. Vorrichtung nach mindestens einem der vorhergehenden Ansprüche, **dadurch gekennzeichnet**, daß die Parameter Maximalkraft, Ruhetonus, Isthaltezeit und die Trainingsparameter "Trainingskraft" und "Trainingshaltezeit" aus der Messung für die Parameterfestlegung automatisch bestimmbar sind.

16. Vorrichtung nach mindestens einem der vorhergehenden Ansprüche, **dadurch gekennzeichnet**, daß das Über- oder Unterschreiten eines Toleranz feldes zum Trainingsabbruch führt.

17. Vorrichtung nach mindestens einem der vorhergehenden Ansprüche, **gekennzeichnet durch** eine logische, optische und akustische Bedienerführung.
